# EUROPÄISCHE PATENTSCHRIFT

(11) **EP 4 603 117 B1**
(45) Veröffentlichungstag und Bekanntmachung des Hinweises auf die Patenterteilung: **25.11.2026**
(21) Anmeldenummer: 24157739.4
(22) Anmeldetag: 15.02.2024
(51) Int. Cl.: A61M 5/142, A61M 39/00, A61M 39/28

(54) **MEDIZINISCHES INFUSIONSSYSTEM UND ZUGEHÖRIGES VERFAHREN**
MEDICAL INFUSION SYSTEM AND ASSOCIATED PROCEDURE
SYSTÈME DE PERFUSION MÉDICALE ET PROCÉDURE ASSOCIÉE

(43) Veröffentlichungstag der Anmeldung: 20.08.2025
(73) Patentinhaber: B. Braun Melsungen AG, 34212 Melsungen (DE)
(72) Erfinder: BARTSCH, Stefan, 36286 Neuenstein (DE)
(74) Vertreter: Tergau & Walkenhorst Intellectual Property GmbH

(56) Entgegenhaltungen:
- CN-U- 202 875 947
- JP-A- 2015 016 200
- US-A1- 2007 112 323
- US-A1- 2016 350 512

## Beschreibung

Die Erfindung betrifft ein medizinisches Infusionssystem mit mindestens einem zum Einlegen in oder Anschließen an eine Infusionspumpe vorgesehenen Einmalartikel, insbesondere einem Infusionsschlauch, sowie mit einer zugehörigen Infusionspumpe, die einen akustischen Signalgeber für eine akustische Signalausgabe aufweist. Die Erfindung betrifft weiterhin ein Verfahren zur Steuerung einer Infusionspumpe mit einer akustischen Signalausgabe.

Infusionspumpen für medizinische Anwendungen sind häufig mit einer akustischen Signalausgabe ausgestattet, die beispielsweise hilfreiche Warn- und Signaltöne ausgeben kann. Eine derartige akustische Signalausgabe kann aber je nach Kontext oder Behandlungsumfeld auch störend sein, vor allem wenn die Lautstärke zu hoch eingestellt ist. Dies kann insbesondere in sensiblen Umgebungen wie Krankenhäusern oder Pflegeeinrichtungen zu Problemen führen, da laute akustische Signale möglicherweise Patienten oder das medizinische Personal stören. Bestehende Infusionspumpen bieten zwar Möglichkeiten, die Lautstärke über eine menügesteuerte Benutzereingabe anzupassen, was jedoch unter Umständen zeitraubend ist. Ein Infusionssystem mit einer derartigen Infusionspumpe ist z.B. aus der JP 2015 016200 A bekannt.

Eine Aufgabe der Erfindung besteht darin, diesbezüglich Abhilfe zu schaffen und ein Infusionssystem der genannten Art anzugeben, das die akustische Signalausgabe auf eine intelligente und anpassungsfähige Weise, insbesondere behandlungsspezifisch, steuert. Des Weiteren soll ein entsprechendes Verfahren angegeben werden.

Die Aufgabe wird erfindungsgemäß gelöst durch ein medizinisches Infusionssystem mit den Merkmalen des Anspruchs 1. Das zugehörige Verfahren ist in Anspruch 10 definiert.

Demnach ist ein medizinisches Infusionssystem vorgesehen mit
- mindestens einem zum Einlegen in oder Anschließen an eine Infusionspumpe vorgesehenen Einmalartikel, der entweder selbst oder durch ein mit ihm verbundenes Zubehörteil mit einem maschinenlesbaren Code versehen ist, welcher eine Solllautstärke einer akustischen Signalausgabe der Infusionspumpe beinhaltet oder repräsentiert,
- mindestens einer Infusionspumpe mit
   ∘ einem akustischen Signalgeber für eine akustische Signalausgabe mit einstellbarer Lautstärke,
   ∘ einer Aufnahme oder einem Anschluss für den Einmalartikel,
   ∘ einem Sensor zur Erfassung des Codes, und
   ∘ einer Pumpensteuerung, die derart ausgelegt oder programmiert ist, dass sie anhand des erfassten Codes die Lautstärke der akustischen Signalausgabe auf die Solllautstärke einstellt.

In einem bevorzugten Anwendungsfeld ist der Einmalartikel oder das Zubehörteil ein Infusionsschlauch oder eine den Fluss von Infusionslösung durch den Infusionsschlauch regulierende Schlauchklemme, insbesondere eine "Anti-Free-Flow-Klemme", die beim Einlegen in die Infusionspumpe durch eine die Aufnahme abdeckende Verschlussklappe oder einen ähnlichen Mechanismus betätigt wird. Die Infusionspumpe ist bevorzugt eine volumetrische Pumpe, insbesondere eine peristaltische Pumpe.

**In** bevorzugter Ausgestaltung ist der Code in Form einer Farbkodierung oder kompletten oder teilweisen Einfärbung auf/an dem Einmalartikel oder dem Zubehörteil ausgebildet, und der zugehörige Sensor ist ein Farbsensor. Vorteilhafterweise ist zum Zweck der Auswertung in der Pumpensteuerung eine Zuordnungstabelle abgespeichert, die die verschiedenen Farben der Farbkodierung mit einer jeweiligen Solllautstärke verknüpft.

**In** bevorzugter Ausgestaltung ist vorgesehen, dass die Infusionspumpe eine graphische Benutzeroberfläche oder Anzeige, insbesondere mit einen Touchdisplay und/oder mit mechanischen Drucktastern oder ähnlichen Bedienelementen, aufweist, die eine manuelle Einstellung der Lautstärke der akustischen Signalausgabe ermöglicht.

Die Pumpensteuerung kann derart konfiguriert sein, dass die mittels des Codes erfasste und eingestellte Solllautstärke eine vorherige manuelle oder werksseitige Lautstärkeeinstellung überschreibt. In diesem Fall ist es möglich, dass die Lautstärke der akustischen Signalausgabe nach dem Entfernen des Einmalartikels wieder auf die vorherige manuelle oder werksseitige Lautstärkeeinstellung zurückgesetzt wird.

In Bezug auf das Verfahren wird die Eingangs genannte Aufgabe gelöst durch ein Verfahren zur Steuerung einer Infusionspumpe mit einer akustischen Signalausgabe, umfassend folgende Schritte:
- Einlegen oder Anschließen eines Einmalartikels an die Infusionspumpe, wobei der Einmalartikel entweder selbst oder durch ein Zubehörteil mit einem maschinenlesbaren Code versehen ist, der die Solllautstärke der akustischen Signalausgabe repräsentiert,
- Erfassen des Codes durch einen Sensor in der Infusionspumpe,
- Ermitteln der Solllautstärke der akustischen Signalausgabe basierend auf dem erfassten Code, und
- Einstellen der Lautstärke der akustischen Signalausgabe der Infusionspumpe entsprechend der ermittelten Solllautstärke durch eine Pumpensteuerung, die derart ausgelegt oder programmiert ist, dass sie anhand des erfassten Codes die Lautstärke der akustischen Signalausgabe auf die Solllautstärke einstellt.

Die zur Vorrichtung genannten Merkmale, Varianten, Aufgaben und Vorteile übertragen sich analog auf das Verfahren und umgekehrt.

Die Erfindung betrifft zusammenfassend insbesondere ein Konzept, bei dem durch die Verwendung eines mit einer Farbkodierung versehenen Einmalartikels, insbesondere einer farblich markierten oder komplett oder teilweise eingefärbten Anti-Free-Flow-Klemme, in Kombination mit einer sensorbasierten Farberkennung im Gerät durch die Infusionspumpe erkannt wird, welche Therapie ausgewählt oder welches Medikament gefördert werden soll und eine entsprechende Lautstärke der akustischen Signalausgabe eingestellt wird. Da Therapien oder Medikamente vermehrt auf bestimmten Stationen eines Krankenhauses benutzt werden, die lautstärkesensitiv sind, kann die Infusionspumpe bereits beim Auswählen einer Therapie oder eines Medikaments die Lautstärke der Hinweis- und Alarmtöne automatisch herabsetzen.

Diese auch als "Automatische Lautstärkeeinstellung" oder kurz "Auto-Volume" bezeichnete Funktion kann vorzugsweise als Option in den Einstellungen der Infusionspumpe ausgewählt (aktivieren/deaktivieren) werden. Die Umsetzung einer solchen "Auto-Volume" Einstellung, kann bei bestehenden Infusionspumpen durch die Pumpensoftware in der Pumpensteuerung erfolgen, es sind keine designtechnischen Anpassungen erforderlich.

**In** einer möglichen Realisierung wird somit werksseitig eine Option "Auto-Volume mittels Farberkennung oder Codeerkennung bei den Lautstärkeeinstellungen einer volumetrischen Pumpe eingefügt, die vom Anwender aktiviert oder deaktiviert werden kann. Bei aktiver Auswahl dieser "Auto-Volume" Option wird beim Einsetzen eines farblich markierten Einmalartikels, speziell einer Anti-Free-Flow-Klemme, je nach erkannter Farbe die Höhe der Lautstärkeeinstellung bzw. der Lautstärkewert automatisch und statisch eingestellt. Beim Entnehmen des Einmalartikels wird die Höhe der Lautstärke wieder auf die werksseitige oder manuell ausgewählte frühere Einstellung zurückgesetzt.

Wie oben angedeutet, lässt sich das Konzept der Farberkennung auf eine Codeerkennung erweitern, insbesondere bei Anbringung von 2D-Codes und/oder 3D-Codes und/oder RFID-Tags mit entsprechendem Informationsgehalt auf/an dem Einmalartikel, wobei in diesem Fall eine Erkennung des Codes durch geeignete Lesegeräte im System erfolgt.

Wie erwähnt werden Therapien oder Medikamente vermehrt auch auf lautstärkesensitiven Station, wie zum Beispiel Intensivstation, Palliativstation oder Operationssaal, ausgewählt oder verabreicht. Die "Auto-Volume" Option unterstützt den Anwender bei der unkomplizierten Auswahl durch eine automatische Lautstärkeeinstellung und reduziert ein "klicken/tappen" durch das Einstellungsmenü bis zu dem die Lautstärkeeinstellungen enthaltenden Untermenü (Zeitreduktion beim Personal). Diese "Auto-Volume" Einstellung hilft auch dem Patienten bei der Genesung im Krankenhaus, da der Schallpegel im Zimmer automatisch angepasst wird.

Verschiedene Ausführungsbeispiele der Erfindung werden nachfolgend anhand der beigefügten Zeichnungen näher erläutert. Es zeigt in jeweils schematischer Weise:
- FIG. 1: einen Abschnitt eines Infusionsschlauchs mit aufgeschobener Schlauchklemme,
- FIG. 2: eine Infusionspumpe mit eingelegtem Infusionsschlauch, und
- FIG. 3: eine als "Free-Flow-Protection-Clamp" ausgebildete Schlauchklemme für einen Infusionsschlauch in Draufsicht.

Gleiche oder gleichartige Elemente sind in allen Figuren mit denselben Bezugszeichen versehen.

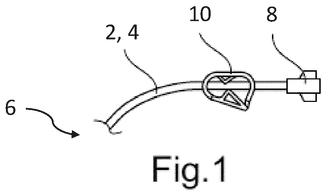

Ein Infusionsschlauch 2, wie er schematisch in FIG. 1 als Beispiel für einen Einmalartikel 4 dargestellt ist, ist ein medizinisches Gerät, das verwendet wird, um Flüssigkeiten wie Medikamente, Elektrolytlösungen oder Nährstoffe direkt in den Körper eines Patienten einzuleiten. Er spielt eine wichtige Rolle bei der intravenösen Therapie, indem er eine Verbindung zwischen der Infusionsquelle, insbesondere einem Infusionsbeutel mit Infusionslösung, und dem Patienten herstellt. Im Zusammenspiel mit einer Infusionspumpe ermöglicht ein Infusionsschlauch 2 eine präzise Dosierung und Verabreichung von Substanzen, um den Gesundheitszustand des Patienten zu verbessern oder aufrechtzuerhalten.

Ein Infusionsschlauch 2 hat zwei Enden, die für unterschiedliche Zwecke ausgelegt sind:
Das Tropfkammer- oder Infusionsnadel-Ende 6: Hier ist üblicherweise eine Tropfkammer angeschlossen, in der die Infusionslösung abtropft und in den Körper des Patienten weiterfließt. Dieses Ende des Infusionsschlauchs 2 ist demnach in der Regel über eine Tropfkammer mit einer Infusionsnadel oder einem Infusionskatheter verbunden, die/der direkt in eine Vene eingeführt wird und somit in den Blutkreislauf des Patienten führt.

Das Infusionsbeutel- oder Infusionspumpen-Ende 8: Dieses Ende des Infusionsschlauchs 2 ist mit einem Infusionsbeutel oder einer Infusionspumpe verbunden. Der Infusionsbeutel oder die Infusionspumpe enthält oder liefert die Infusionslösung und reguliert den Fluss durch den Infusionsschlauch 2 in die Tropfkammer.

Zusammenfassend erfüllen die beiden Enden eines Infusionsschlauchs 2 unterschiedliche Funktionen: Das eine Ende ist mit der Infusionsquelle verbunden, während das andere Ende den direkten Zugang zum Patienten ermöglicht, um die Infusionsflüssigkeit in den Körper zu leiten.

Eine Schlauchklemme 10, wie sie in FIG. 1 schematisch angedeutet ist, ist ein wichtiges Zubehörteil im Zusammenhang mit einem Infusionsschlauch 2. Sie wird verwendet, um den Fluss der Infusionslösung im Infusionsschlauch 2 manuell zu regulieren oder ganz zu stoppen. Die Schlauchklemme 10 besteht normalerweise aus Kunststoff und wird auf den Infusionsschlauch 2 geschoben, um ihn bedarfsweise zu quetschen und dadurch den Fluss der Flüssigkeit zu kontrollieren.

Mit anderen Worten besteht die Funktion einer Schlauchklemme 10 darin, den Infusionsfluss zu regulieren, indem sie - je nach Stellung - den Infusionsschlauch 2 gar nicht, teilweise oder vollständig zusammendrückt. Das kann nützlich sein, um den Fluss zu unterbrechen, wenn zum Beispiel die Infusion gestoppt werden muss, oder um die Rate der Infusion anzupassen, indem der Infusionsschlauch 2 teilweise geöffnet oder geschlossen wird.

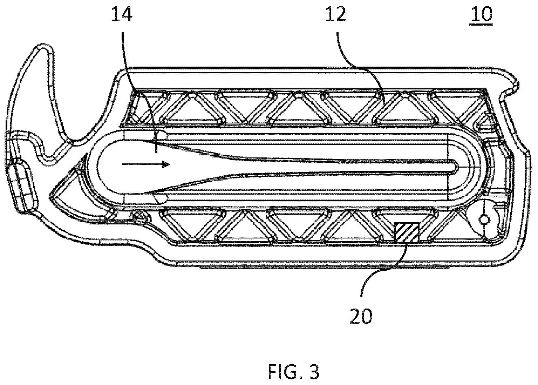

Eine Variante einer Schlauchklemme 10 ist in FIG. 3 beispielhaft in detaillierter Draufsicht dargestellt. Die Schlauchklemme 10 weist einen flachen Grundkörper oder Rahmen 12, typischerweise aus Kunststoff, auf, welcher ein sich hier von rechts nach links verbreiterndes Langloch 14 umschließt. Die Schlauchklemme 10 ist im Betrieb auf den Infusionsschlauch 2 geschoben und umschließt ihn, so dass der Infusionsschlauch 2 das Langloch 14 durchquert. Wenn der Infusionsschlauch 2 im breiten Ende des Langlochs 14 angeordnet ist, wird er nicht gequetscht und hat seinen maximalen Querschnitt (Öffnungsstellung). Wird der Infusionsschlauch 2 hingegen relativ zur Schlauchklemme 10 in Pfeilrichtung zum schmalen Ende des Langlochs 14 hin verschoben, wird er zunehmend gequetscht und schließlich komplett verschlossen (Schließstellung).

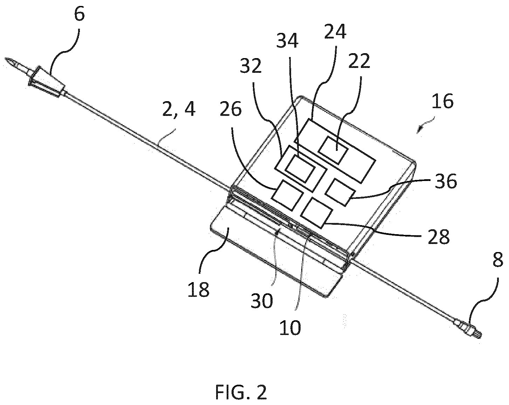

Ein Infusionsschlauch 2 der genannten Art kann insbesondere zum Einlegen in eine als peristaltische Pumpe ausgebildete Infusionspumpe 16 ausgelegt sein, wie in FIG. 2 schematisch dargestellt. Bei einer peristaltischen Pumpe wird der Infusionsschlauch 2 periodisch durch rotierende Walzen oder Rollen zusammengedrückt und entspannt, um die Flüssigkeit zu fördern. Der Vorteil einer derartigen Pumpe liegt darin, dass die Flüssigkeit im Schlauch bleibt und nicht mit der Pumpenmechanik in Berührung kommt, was die Kontamination der Flüssigkeit verhindert.

Ferner kann eine auf den Infusionsschlauch 2 oder kurz Schlauch aufgeschobene Schlauchklemme 10 als sogenannte "Anti-Free-Flow-Klemme" oder "Free-Flow-Protection-Clamp" ausgebildet sein. Das ist eine zweite Sicherheitsklemme oder kurz Klemme, die einen freien Fluss (Free-Flow) der zu fördernden Flüssigkeit bei der Entnahme aus dem Gerät (d. h. der Infusionspumpe) verhindern soll. Im Auslieferzustand des Schlauchsets ist die Klemme geöffnet. Diese muss nämlich während der Lagerung geöffnet sein, da sich durch längere Lagerung der Schlauch deformieren kann oder der Kunststoffeinmalartikel plastisch verformt. Beim Einlegen in das Gerät ist die Klemme daher zunächst geöffnet, wird aber durch das Schließen einer Klappe zugeschoben und nach bestandenem Okklusionstest wieder durch das Gerät geöffnet. Beim Entnehmen des Einmalartikels aus dem Gerät, zieht die Klappe per Öse, die in einen Haken der Klemme eingehakt wird, die Klemme wieder zu und schließt den Schlauch gegen Free-Flow. Deshalb wird die Klemme auch Free-Flow-Protection-Clamp genannt.

Zu diesem Zweck kann die Infusionspumpe 16 eine passend geformte, durch eine Verschlussklappe 18 verschließbare Aufnahme 30 (in FIG. 2 durch die Verschlussklappe 30 verdeckt) für den Infusionsschlauch 2 und die Schlauchklemme 10 aufweisen, wobei durch das Auf- und Zuklappen der Verschlussklappe 18 (alternativ durch das Einlegen oder Entnehmen des Infusionsschlauchs 2 mit der Schlauchklemme 10) automatisch die Schlauchklemme 10 angesteuert wird, wie oben beschrieben.

Der Infusionsschlauch 2 mit bereits aufgesetzter Schlauchklemme 10 wird normalerweise als vormontierte Einheit im sterilisierten Zustand in einer sterilen bzw. hermetisch verschlossenen Verpackung aufbewahrt und zur Verwendung vorgehalten. Die Verpackung kann ferner einen zugehörigen Infusionsbeutel mit Infusionslösung für eine spezifische Anwendung oder Therapie enthalten, so dass insgesamt ein Set enthaltend Infusionsschlauch 2, Schlauchklemme 10 und Infusionsbeutel (nicht dargestellt) bereitgestellt ist.

Die Infusionspumpe 16 weist eine integrierte Pumpensteuerung 24 auf, die über eine Benutzeroberfläche ansprechbar und konfigurierbar / programmierbar ist. Beispielsweise kann die Benutzeroberfläche 32 ein Touchdisplay 34 zur visuellen Darstellung von den Pumpenbetrieb betreffenden Informationen und zur Eingabe / Programmierung von Einstellungen, Parametern und dergleichen aufweisen. Typischerweise lassen sich verschiedene Konfigurationen, Einstellungen oder Programme über eine integrierte Menüführung erreichen, anzeigen und auch verändern. Die Menüführung wird beispielsweise durch ein in die Pumpensteuerung 24 integriertes oder mit dieser kommunizierendes Betriebssystem angesteuert. Alternativ können Anzeige- und Bedienelemente auch getrennt sein, etwa in Gestalt eines Displays und separater physischer Schalter, Taster oder sonstiger Bedienelemente. Auch gemischte Konzepte aus Touchdisplay 34 und zusätzlichen Bedienelementen sind möglich.

Neben einer visuellen Darstellung von Informationen kann die Infusionspumpe 16 auch eine Ausgabe akustischer Signale mittels eines akustischen Signalgebers 36, insbesondere eines Lautsprechers, aufweisen. Somit kann zum Beispiel ein akustisches Feedback zur Bedienung oder zum Betrieb in Form von Betätigungs-, Hinweis- oder Warntönen (oder durch Sprachausgabe) erfolgen.

Alternativ und/oder zusätzlich zu einem Lautsprecher kann ein Körperschallwandler / Exciter als akustischer Signalgeber zum Einsatz kommen. Ein Körperschallwandler ist ein Gerät, das Schall erzeugt, indem es Vibrationen direkt auf eine Oberfläche überträgt, anstatt Schall durch die Luft zu verbreiten. Mit anderen Worten überträgt ein Exciter Schall durch direkten Kontakt mit einem festen Objekt.

Der akustische Signalgeber 36 wird vorzugsweise ebenfalls über die Pumpensteuerung 24 oder ein zugehöriges Betriebssystem angesteuert und ist zweckmäßigerweise hinsichtlich seiner Lautstärke einstellbar. Die Lautstärkeeinstellungen sind dann zum Beispiel über ein entsprechendes Untermenü der Menüführung erreichbar und veränderbar. Das heißt aber auch, dass der Benutzer oder Bediener in der Regel erst in dieses Untermenü navigieren oder "klicken/tappen" muss, um die Lautstärke zu ändern. In einem lautstärkesensitiven Umfeld wie zum Beispiel einer Intensivstation, einer Palliativstation oder einem Operationssaal einer medizinischen Behandlungseinrichtung, etwa einem Krankenhaus, kann dies zu unerwünschter Lärmbelästigung und zur Ablenkung durch dem entgegenwirkende Bedieneingriffe führen.

Um dies zu verhindern, ist in einer bevorzugten Ausgestaltung die mit dem Infusionsschlauch 2 verbundene oder verbindbare und im oben beschriebenen Sinne als "Anti-Free-Flow-Klemme" zur Wechselwirkung mit einer zugehörigen Infusionspumpe 16 ausgebildete Schlauchklemme 10 an einer vorbestimmten Stelle mit einem maschinenlesbaren Code versehen / ausgestattet / beschriftet oder ausgebildet, der eine gewünschte Lautstärke oder Solllautstärke der akustischen Signalausgabe enthält oder repräsentiert. Dieser Code wird von der Infusionspumpe 16 durch deren Pumpensteuerung 24 oder Betriebssystem beim Einlegen oder Anschließen des Infusionsschlauchs 2 ausgelesen bzw. sensorisch erfasst und in eine automatische Einstellung oder Anpassung der Lautstärke der akustischen Signalausgabe durch den akustischen Signalgeber 36 umgesetzt. Das heißt im Ergebnis, dass die Lautstärke automatisch im Einklang mit einer medizinischen Behandlung oder Therapie eingestellt wird, die mit dem jeweiligen Infusionsschlauch 2 verknüpft ist - etwa dadurch, dass der Infusionsschlauch 2 Teil eines Sets mit einem zugehörigen, behandlungsspezifischen Infusionsbeutel ist. Das Set kann beispielsweise auch nur aus einem Schlauch, einer Schlauchklemme und einer Tropfkammer mit Anstechvorrichtung an eine Infusionsflasche ausgebildet sein.

Ein Code ist im Allgemeinen eine systematische Anordnung von Zeichen, Symbolen, Farben, Signalen oder anderen Informationsträgern, die dazu dienen, Informationen zu speichern, zu übermitteln, zu verschlüsseln, zu entschlüsseln oder zu verbergen. Ein Code ermöglicht die Speicherung und Übertragung von Informationen in einer strukturierten und/oder kodierten (insbesondere verschlüsselten) Form.

In bevorzugter Ausgestaltung handelt es sich bei dem Code um eine Farbkodierung, etwa in Gestalt einer (insbesondere einfarbigen) Farbmarkierung oder eines (insbesondere mehrfarbigen) Farbmusters an einer vorbestimmten Stelle, zum Beispiel am Rahmen der Schlauchklemme 10. Die zur Kodierung und Kennzeichnung der Schlauchklemme 10 verwendete Farbe kann beispielsweise als Farbaufdruck, als Beschichtung, durch Anbringung eines Farbelements oder durch geeignete Wahl des Grundmaterials erzeugt sein. Die Schlauchklemme 10 kann in ihrer Gesamtheit oder lediglich in einem Messbereich entsprechend eingefärbt sein.

Dabei ist in einer zugehörigen Zuordnungstabelle 22 (Lookup- bzw. Mapping-tabelle) oder Farbtabelle eine idealerweise eineindeutige Zuordnung zwischen Farbe und Lautstärke der akustischen Signalausgabe definiert, wobei zur Vereinfachung und zur Reduktion der notwendigen unterscheidbaren Farben bestimmte Laustärkepegel oder -stufen gebildet sein können; etwa von 1 (still oder sehr leise) bis 9 (sehr laut).

Die Infusionspumpe 16, in die der Infusionsschlauch 2 mit der Schlauchklemme 10 zur Benutzung eingelegt wird, ist mit Mitteln für eine automatisierte, maschinelle Erfassung und Dekodierung / Entschlüsselung des auf der Schlauchklemme 10 angebrachten bzw. angeordneten Codes ausgestattet. Unter Verwendung der oben erwähnten Zuordnungstabelle 22, die vorzugsweise in einer Speichereinheit der Pumpensteuerung 24 hinterlegt bzw. abgespeichert ist, ermittelt ein in der Pumpensteuerung 24 implementierter Algorithmus aus der im Code enthaltenen Information die gewünschte Laustärkeeinstellung und stellt sie automatisch ein. Ein zuvor (per Werkseinstellung oder Benutzereingabe) eingestellter anderer Lautstärkewert wird dabei überschrieben. Der im zugehörigen Untermenü der Menüführung angezeigte Wert wird auf den aktuellen Wert angepasst.

Die beschriebene Überprüfung und Einstellung der Lautstärke kann beispielsweise immer nach einem Einlegen oder Anschließen eines Infusionsschlauchs 2 in/an die Infusionspumpe 16 durchgeführt werden, wenn ein zum Beispiel ein Sensor die Anwesenheit der Schlauchklemme 10 in der Aufnahme 32 oder am Anschluss erkennt. Je nach vorgegebener und gegebenenfalls einstellbarer Systemeinstellung kann die durch den erfassten Code vorgegebene Lautstärkeeinstellung während der Anwesenheit des Infusionsschlauchs 2 in der Infusionspumpe 16 permanent (statisch) und somit unveränderbar oder manuell überschreibbar sein. Denkbar ist auch eine auswählbare Systemeinstellung, die die beschriebene automatische Lautstärkeregelung deaktiviert. Es kann auch vorgesehen sein, dass ein nur autorisierter Benutzer, etwa nach Eingabe eines Berechtigungscodes über ein Tastenfeld oder nach sonstiger Autorisierung, in der Lage ist, derartige Systemeinstellungen oder die Lautstärke zu ändern. Des Weiteren kann vorgesehen sein, dass die Lautstärke der akustischen Signalausgabe nach einer Entnahme des Infusionsschlauchs 2 aus der Infusionspumpe 16 automatisch wieder auf den früheren Wert zurückgesetzt wird.

Zur Erkennung der Farbe der Farbkodierung 20 ist zweckmäßigerweise ein optischer Sensor, insbesondere ein Farbsensor 26 mit zugehöriger Beleuchtungseinheit 28 in die Infusionspumpe 16 integriert. Beispielsweise können die Komponenten derart angeordnet sein, dass von der Beleuchtungseinheit 28 emittiertes Licht auf die in der Aufnahme 30 sitzende Schlauchklemme 10 fällt und von dieser reflektiert wird, wobei das reflektierte Licht auf den Farbsensor 16 fällt. Der Farbsensor wertet dann beispielsweise ein Reflektionsspektrum aus. Alternativ kann bei entsprechender Anordnung der Komponenten ein Transmissionsspektrum ausgewertet werden, sofern das die Farbkodierung 20 tragende Element hinreichend lichtdurchlässig ist. In beiden Fällen (Reflektion oder Transmission) kann im Prinzip anstelle einer Beleuchtung durch eine Beleuchtungseinheit 28 eine Beleuchtung durch Umgebungslicht erfolgen, was jedoch wegen der schwankenden Umgebungslichtverhältnisse nicht immer praktikabel und zumindest fehleranfälliger ist.

Anstelle von und/oder zusätzlich zu einer herkömmlichen Farbe im sichtbaren Bereich des optischen Spektrums kann auch eine spektrale Eigenschaft eines Emissionsspektrums und/oder Transmissionsspektrums im nicht-sichtbaren Spektralbereich zur Kennzeichnung der Schlauchklemme 10 und zur Kodierung der Lautstärkeeinstellung verwendet werden. Dementsprechend kann die Beleuchtungseinheit 28 beispielsweise für die Emission von IR- oder UV-Licht ausgelegt sein. Entsprechendes gilt für die spektrale Sensitivität des Farbsensors 26, die an die interessierende Wellenlänge angepasst ist. Auch die Anregung und Auswertung von Fluoreszenzeffekten als Träger der Kodierung ist denkbar.

Der Farbsensor 26 kann so beschaffen sein, dass er die Farbe der Farbkodierung 20 ermittelt und dem Auswertungsalgorithmus der Pumpensteuerung 24 mitteilt. Es kann aber auch so sein, dass nur gewisse physikalische Eigenschaften des reflektierten oder transmittierten Lichts durch den Farbsensor 26 gemessen werden, wobei die eigentliche Auswertung und Bestimmung der Farbe dann in der Pumpensteuerung 24 erfolgt. Anstelle einer in die Infusionspumpe 16 integrierten Pumpensteuerung 24 kann auch eine Ausgestaltung vorgesehen sein, bei der die entsprechende Funktionalität zumindest teilweise in eine externe Komponente ausgelagert ist und beispielsweise durch einen Zentralrechner oder Cloudservice bereitgestellt wird.

In einer Abwandlung des beschriebenen Konzepts kann die zur Festlegung der Lautstärke der akustischen Signalausgabe verwendete Farbkodierung 20 an anderer Stelle als auf/an einer Schlauchklemme 10 angeordnet sein. Möglich ist beispielsweise eine mit dem Infusionsschlauch 2 verbundene oder verbindbare Markierungsfahne oder sonstige Kennzeichnung oder ein anderes entsprechendes Zubehörteil. Des Weiteren ist ein Farbaufdruck oder eine Farbschicht oder eine materialbedingte Farbkodierung direkt auf der Oberfläche des Infusionsschlauchs 2 eine mögliche Alternative. Das heißt, der Infusionsschlauch 2 trägt dann selbst die Farbkodierung 20.

Anstelle einer Farbkodierung 20 lassen sich auch auf dem Infusionsschlauch 2, der Schlauchklemme 10 oder einem anderen Zubehörteil aufgedruckte oder anderweitige angebrachte oder angeordnete Zeichencodes verwenden, die durch die Infusionspumpe 16 über einen optischen Sensor lesbar / auswertbar sind und damit die oben beschriebene Funktion abbilden. Generell bezieht sich der Begriff "Zeichencode" auf jede Art von kodierter Darstellung von Informationen, die auf visuellen Elementen basiert, sei es durch spezielle Symbole, grafische Muster oder andere visuelle Markierungen. Insbesondere kommen Barcodes und QR-Codes oder generell 2D-Codes oder 3D-Codes als Informationsträger in Betracht: Dies sind Arten von Zeichencodes, die visuelle Muster verwenden, um Informationen darzustellen. Barcodes bestehen aus einer Reihe von parallelen Linien unterschiedlicher Breite, während QR-Codes typischerweise aus einem Muster von quadratischen Blöcken bestehen. Beide werden durch sensorbasierte Lesegeräte erfasst und in digitale Daten umgewandelt.

Als weitere Alternative lassen sich RFID-Tags, welche die mit dem Infusionsschlauch 2 assoziierte Lautstärkeeinstellung in kodierter Form enthalten, in den Infusionsschlauch 2, die Schlauchklemme 10 oder das sonstige Zubehörteil integrieren. Die auf dem RFID-Tag hinterlegten Informationen lassen sich durch einen geeigneten Sensor der Infusionspumpe 16 auslesen und, wie oben beschrieben, auswerten und nutzen.

Anstelle eines Infusionsschlauchs 2 kann als Einmalartikel 4 auch eine medizinische Spritze zum Einlegen in die Infusionspumpe 16, die in diesem Fall als Spritzenpumpe ausgestaltet ist, vorgesehen und entsprechend mit einer die Lautstärke der Signalausgabe ansteuernden Farbkodierung 20 oder sonstigen Codierung ausgestattet sein. Das heißt, alle weiter oben am Beispiel einer peristaltischen Pumpe beschriebenen Konzepte zur automatischen Lautstärkeeistellung mittels codierter Schlauchklemme und zugehöriger Erfassung lassen sich sinngemäß auf eine Spritzenpumpe übertragen, wobei in diesem Fall vorzugsweise eine entsprechend codierte Spritze die Ansteuerung bewirkt.

Eine andere Bezeichnung für eine Spritzenpumpe ist eine Kolbenpumpe. Im Gegensatz zur peristaltischen Pumpe, die durch Quetschen und Freigeben eines flexiblen Schlauches arbeitet, verwendet die Spritzenpumpe einen Kolbenmechanismus (innerhalb der Spritze), um Flüssigkeit zu fördern. Dabei bewegt sich ein Kolben in einem Zylinder vor und zurück, um Flüssigkeit anzusaugen und zu fördern.

### Bezugszeichenliste

- 2: Infusionsschlauch
- 4: Einmalartikel
- 6: Tropfkammer- oder Infusionsnadel-Ende
- 8: Infusionsbeutel- oder Infusionspumpen-Ende
- 10: Schlauchklemme
- 12: Rahmen
- 14: Langloch
- 16: Infusionspumpe
- 18: Verschlussklappe
- 20: Farbkodierung
- 22: Zuordnungstabelle
- 24: Pumpensteuerung
- 26: Farbsensor
- 28: Beleuchtungseinheit
- 30: Aufnahme
- 32: Benutzeroberfläche
- 34: Touchdisplay
- 36: Akustischer Signalgeber

## Patentansprüche

1. Medizinisches Infusionssystem mit
• mindestens einem zum Einlegen in oder Anschließen an eine Infusionspumpe (16) vorgesehenen Einmalartikel (4), der entweder selbst oder durch ein mit ihm verbundenes Zubehörteil mit einem maschinenlesbaren Code versehen ist, welcher eine Solllautstärke einer akustischen Signalausgabe der Infusionspumpe (16) beinhaltet oder repräsentiert,
• mindestens einer Infusionspumpe (16) mit
∘ einem akustischen Signalgeber (36) für eine akustische Signalausgabe mit einstellbarer Lautstärke,
∘ einer Aufnahme (30) oder einem Anschluss für den Einmalartikel (4),
∘ einem Sensor zur Erfassung des Codes, und
∘ einer Pumpensteuerung (24), die derart ausgelegt oder programmiert ist, dass sie anhand des erfassten Codes die Lautstärke der akustischen Signalausgabe auf die Solllautstärke einstellt.

2. Infusionssystem nach Anspruch 1, wobei der Einmalartikel (4) oder das Zubehörteil ein Infusionsschlauch (2) oder eine Schlauchklemme (10) oder eine medizinische Spritze ist.

3. Infusionssystem nach einem der vorangehenden Ansprüche, wobei die Infusionspumpe (16) eine volumetrische Pumpe, insbesondere eine peristaltische Pumpe, ist.

4. Infusionssystem nach einem der vorangehenden Ansprüche, wobei der Code in Form einer Farbkodierung (20) oder einer kompletten oder teilweisen Einfärbung des Einmalartikels (4) ausgebildet ist.

5. Infusionssystem nach Anspruch 4, wobei der Sensor ein Farbsensor (26) ist.

6. Infusionssystem nach Anspruch 4 oder 5, wobei in der Pumpensteuerung (24) eine Zuordnungstabelle (22) abgespeichert ist, die Farben der Farbkodierung (20) mit einer jeweils zugehörigen Solllautstärke verknüpft.

7. Infusionssystem nach einem der vorangehenden Ansprüche, wobei die Infusionspumpe (16) eine Benutzeroberfläche (32), insbesondere mit einen Touchdisplay (34), aufweist, die eine manuelle Einstellung der Lautstärke der akustischen Signalausgabe ermöglicht.

8. Infusionssystem nach einem der vorangehenden Ansprüche, wobei die Pumpensteuerung (24) derart konfiguriert ist, dass die mittels des Codes erfasste und eingestellte Solllautstärke eine vorherige manuelle oder werksseitige Lautstärkeeinstellung überschreibt.

9. Infusionssystem nach Anspruch 8, wobei die Pumpensteuerung (24) derart konfiguriert ist, dass die Lautstärke der akustischen Signalausgabe nach dem Entfernen des Einmalartikels (4) wieder auf die vorherige manuelle oder werksseitige Lautstärkeeinstellung zurückgesetzt wird.

10. Verfahren zur Steuerung einer Infusionspumpe (16) mit einer akustischen Signalausgabe, umfassend folgende Schritte:
• Einlegen oder Anschließen eines Einmalartikels (4) an die Infusionspumpe (16), wobei der Einmalartikel (4) entweder selbst oder durch ein Zubehörteil mit einem maschinenlesbaren Code versehen ist, der die Solllautstärke der akustischen Signalausgabe repräsentiert,
• Erfassen des Codes durch einen Sensor in der Infusionspumpe (16),
• Ermitteln der Solllautstärke der akustischen Signalausgabe basierend auf dem erfassten Code, und
• Einstellen der Lautstärke der akustischen Signalausgabe der Infusionspumpe (16) entsprechend der ermittelten Solllautstärke mittels einer Pumpensteuerung (24), die derart ausgelegt oder programmiert ist, dass sie anhand des erfassten Codes die Lautstärke der akustischen Signalausgabe auf die Solllautstärke einstellt.

## Claims

1. Medical infusion system with
- at least one disposable article (4) which is intended for insertion into or connection to an infusion pump (16) and which itself or by way of an accessory connected to it, is provided with a machine-readable code which contains or represents a nominal volume of an acoustic signal output by the infusion pump (16),
- at least one acoustic infusion pump (16) with
- an acoustic signal transmitter (36) for an acoustic signal output with an adjustable volume,
- a receiver (30) or a connection for the disposable article (4),
- a sensor for recording the code, and
- a pump control (24) which is designed or programmed in such a way that by way of the recorded code, it adjusts the volume of the acoustic signal output to the nominal volume.

2. Infusion system according to claim 1, wherein the disposable article (4) or the accessory is an infusion tube (2) or a tube clamp (10) or a medical syringe.

3. Infusion system according to any one of the preceding claims, wherein the infusion pump (16) is a volumetric pump, more particularly a peristaltic pump.

4. Infusion system according to any one of the preceding claims, wherein the code is in the form of colour coding (20) or complete of partial colouring of the disposable article (4).

5. Infusion system according to claim 4, wherein the sensor is a colour sensor (26).

6. Infusion system according to claim 4 or 5, wherein an allocation table (22) is stored in the pump control (24) which links the colours of the colour coding (20) to a respectively associated nominal volume.

7. Infusion system according to any one of the preceding claims, wherein the infusion pump (16) comprises a user interface (32), more particularly with a touch display (34), which allows manual adjustment of the volume of the acoustic signal output.

8. Infusion system according to any one of the preceding claims, wherein the pump control (24) is configured so that nominal volume recorded and adjusted by the code overwrites a previous manually or factory-set volume setting.

9. Infusion system according to claim 8, wherein the pump control (24) is configured so that after removal of the disposable article (4), the volume of the acoustic signal output is reset to the previous manual or factory-set volume setting.

10. Method of controlling an infusion pump (16) with an acoustic signal output, comprising the following steps:
- insertion into or connection of a disposable article (4) to the infusion pump (16), wherein the disposable article (4) is either itself, or by way of an accessory, provided with a machine-readable code that represents the nominal volume of the acoustic signal output,
- recording of the code by a sensor in the infusion pump (16),
- determination of the nominal volume of the acoustic signal output based on the recorded code, and
- adjustment of the volume of the acoustic signal output of the infusion pump (16) in accordance with the determined nominal volume by way of a pump control (24), which is designed or programmed in such a way that based on the recorded code it adjusts the volume of the acoustic signal output to the nominal volume.

## Revendications

1. Système de perfusion médical avec
• au moins un article à usage unique (4) prévu pour insertion dans ou raccordement à une pompe de perfusion (16), qui est doté d'un code lisible par une machine, soit lui-même, soit par une partie accessoire reliée à celui-ci, lequel contient ou représente une intensité sonore théorique d'une émission acoustique de signaux de la pompe de perfusion (16),
• au moins une pompe de perfusion (16) avec
∘ un émetteur de signaux acoustique (36) pour une émission acoustique de signaux avec une intensité sonore réglable,
∘ un logement (30) ou un raccord pour l'article à usage unique (4),
∘ un capteur pour la saisie du code, et
∘ une commande de pompe (24), qui est conçue ou programmée de telle manière qu'elle règle l'intensité sonore de l'émission acoustique de signaux à l'intensité sonore théorique à l'aide du code saisi.

2. Système de perfusion selon la revendication 1, sachant que l'article à usage unique (4) ou la partie accessoire est un flexible de perfusion (2) ou un collier de serrage de flexible (10) ou une seringue médicale.

3. Système de perfusion selon l'une quelconque des revendications précédentes, sachant que la pompe de perfusion (16) est une pompe volumétrique, en particulier une pompe péristaltique.

4. Système de perfusion selon l'une quelconque des revendications précédentes, sachant que le code est constitué sous la forme d'une codification en couleurs (20) ou d'une coloration complète ou partielle de l'article à usage unique (4).

5. Système de perfusion selon la revendication 4, sachant que le capteur est un capteur de couleur (26).

6. Système de perfusion selon la revendication 4 ou 5, sachant qu'un tableau d'attribution (22) est mémorisé dans la commande de pompe (24), qui associe les couleurs de la codification de couleur (20) à une intensité sonore théorique respectivement associée.

7. Système de perfusion selon l'une quelconque des revendications précédentes, sachant que la pompe de perfusion (16) comporte une surface utilisateur (32), en particulier avec un affichage tactile (34), qui permet un réglage manuel de l'intensité sonore de l'émission acoustique de signaux.

8. Système de perfusion selon l'une quelconque des revendications précédentes, sachant que la commande de pompe (24) est configurée de telle manière que l'intensité sonore théorique saisie et réglée au moyen du code écrase un réglage d'intensité sonore manuel ou d'usine précédent.

9. Système de perfusion selon la revendication 8, sachant que la commande de pompe (24) est configurée de telle manière que l'intensité sonore de l'émission acoustique de signaux est réinitialisée après enlèvement de l'article à usage unique (4) à nouveau au réglage d'intensité sonore manuel ou d'usine précédent.

10. Procédé de commande d'une pompe de perfusion (16) avec une émission acoustique de signaux, comprenant les étapes suivantes :
• insertion ou raccordement d'un article à usage unique (4) à la pompe de perfusion (16), sachant que l'article à usage unique (4) est doté d'un code lisible par une machine, soit lui-même, soit par une partie accessoire, qui représente l'intensité sonore théorique de l'émission acoustique de signaux,
• saisie du code par un capteur dans la pompe de perfusion (16),
• détermination de l'intensité sonore théorique de l'émission acoustique de signaux en se basant sur le code saisi, et
• réglage de l'intensité sonore de l'émission acoustique de signaux de la pompe de perfusion (16) corresponndant à l'intensité sonore théorique déterminée au moyen d'une commande de pompe (24), qui est conçue ou programmée de telle manière qu'elle règle l'intensité sonore de l'émission acoustique de signaux à l'intensité sonore théorique à l'aide du code saisi.
